(19) Europäisches Patentamt
European Patent Office
Office européen des brevets

(11) **EP 2 445 387 B1**

(12) **EUROPÄISCHE PATENTSCHRIFT**

(45) Veröffentlichungstag und Bekanntmachung des Hinweises auf die Patenterteilung:
**16.12.2015 Patentblatt 2015/51**

(21) Anmeldenummer: **10724537.5**

(22) Anmeldetag: **22.06.2010**

(51) Int Cl.:
***A61B 3/113*** *(2006.01)*

(86) Internationale Anmeldenummer:
**PCT/EP2010/058854**

(87) Internationale Veröffentlichungsnummer:
**WO 2010/149672 (29.12.2010 Gazette 2010/52)**

(54) **FIXATIONSKONTROLLEINRICHTUNG UND VERFAHREN ZUR KONTROLLE EINER FIXATION EINES AUGES**

FIXATION CONTROL DEVICE AND METHOD FOR CONTROLLING THE FIXATION OF AN EYE

DISPOSITIF DE CONTRÔLE DE FIXATION ET PROCÉDÉ DESTINÉ AU CONTRÔLE DE LA FIXATION D'UN OEIL

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **23.06.2009 DE 102009030465**

(43) Veröffentlichungstag der Anmeldung:
**02.05.2012 Patentblatt 2012/18**

(73) Patentinhaber: **Carl Zeiss Meditec AG**
**07745 Jena (DE)**

(72) Erfinder:
• **BERGT, Michael**
**99425 Weimar (DE)**
• **STOBRAWA, Gregor**
**07743 Jena (DE)**
• **SLUYTERMAN VAN LANGEWEYDE, Georg**
**07749 Jena (DE)**
• **BISCHOFF, Mark**
**07749 Jena (DE)**

(74) Vertreter: **Beck, Bernard**
**Carl Zeiss AG**
**c/o Patentabteilung Jena**
**Carl-Zeiss-Promenade 10**
**07745 Jena (DE)**

(56) Entgegenhaltungen:
**US-A1- 2004 252 277 US-A1- 2008 084 539**

**Beschreibung**

Die Erfindung betrifft eine Fixationskontrolleinrichtung für ein ophthalmologisches Gerät, aufweisend eine Fixierlichtquelle für sichtbares Licht und eine Optik zur Abbildung der Fixierlichtquelle auf einen Augenhintergrund, und ein Verfahren zur Kontrolle einer Fixation eines Auges.

Bei einigen ophthalmologischen Geräten ist es notwendig, dass der zu untersuchende oder zu behandelnde Patient in eine definierte Richtung blickt. Diese Ausrichtung ist in der Regel maßgeblich für die Korrektheit der Messung beziehungsweise der Therapie und mithin für den Behandlungserfolg. Dies gilt insbesondere für Refraktometer, Wellenfrontaberrometer und refraktive Laser.

Üblicherweise wird im Sehfeld des Patienten ein sichtbares Fixier-Ziel bereitgestellt, das der Patient visuell fixieren soll. Ohne weitere Maßnahmen müsste der Bediener des Geräts, also der Untersucher beziehungsweise der Behandler, darauf vertrauen, dass der Patient sich an diese Anweisung hält. Insbesondere über längere Zeiträume hinweg nimmt jedoch die Wahrscheinlichkeit zu, dass der Patient den Zustand der Fixation verlässt. Das menschliche Auge kann insbesondere sogenannte Sakkadenbewegungen mit einer Geschwindigkeit bis zu etwa 600° pro Sekunde durchführen, so dass drastische Abweichungen der Blickrichtung innerhalb kurzer Zeit auftreten können. Streuungen in Diagnose- oder Therapieergebnissen resultieren oft aus der Unsicherheit in der Fixation. Besonders problematisch ist die Einhaltung und Überwachung der Fixation bei Kindern, Blinden und anderweitig beeinträchtigten Personen. Der Bediener benötigt also eine möglichst objektive Information darüber, ob der Patient das Fixier-Ziel tatsächlich fixiert und wann dies gegebenenfalls nicht mehr zutrifft.

Zu diesem Zweck sind im Stand der Technik unterschiedliche Ansätze bekannt. So beschreibt US 2006/0142742 A1 ein ophthalmologisches Behandlungsgerät mit einem UV-Laser, bei dem ein zusätzlicher Lichtstrahl, der auf der Retina des Patienten einen Lichtfleck erzeugt, zur visuellen Fixation bereitgestellt wird. Mit einer Kamera wird ein Bild einer Ebene der Retina im Bereich der Fovea centralis (nachfolgend verkürzt als Fovea bezeichnet) aufgenommen. Zur Überwachung der Fixation wird anhand dieses Bildes geprüft, ob der Lichtfleck auf der Fovea liegt. Zusätzlich kann ein Bild der Pupille aufgenommen werden, um anhand des Schwerpunkts der Pupille oder der relativen Lage des Lichtflecks zur Pupille die Fixation zu überwachen. Diese Art der Fixationskontrolle hat mehrere Nachteile. Zunächst ist zwangsläufig ein zweidimensionaler Bildsensor mit großer Pixelanzahl erforderlich, um eine ausreichende Genauigkeit der Überwachung zu erreichen. In der Folge ist die Frequenz der Bildaufnahme nach oben hin beschränkt, da zum einen das Auslesen des Sensors langwierig ist und zum anderen das anschließende Auswerten zumindest von Bildteilen aufgrund der Datenmenge relativ lange dauert. Daraus resultiert eine relative langsame Reaktion auf einen Verlust der Fixation mit entsprechenden Gefahren bei einer schnellen Augenbewegung.

In US 6,027,216 wird ein Verfahren zur Überwachung der Fixation beschrieben, bei der Augenhintergrund beleuchtet und von dort rückgestreute Strahlung mit einem polarisationsempfindlichen Detektor aufgenommen wird. Anhand von Veränderungen der Polarisation zwischen dem Beleuchtungslicht und dem rückgestreuten Licht wird ermittelt, ob die Rückstreuung an der Fovea oder am Rest der Retina erfolgt ist. Die Rückstreuung an der Fovea wird als Indikator für die visuelle Fixation durch den Patienten genutzt. Nachteilig ist, dass die polarisationsempfindliche Detektion aufwendig ist. Zudem sind die Veränderungen an der Polarisation gering gegenüber unerwünschten Einflüssen wie der Depolarisation in Cornea und Augenlinse, so dass die Unterscheidung zwischen der Rückstreuung an der Fovea und dem Rest der Retina mit relativ großer Unsicherheit behaftet ist.

Aus US 2004/252277 A1 ist ein Gerät zum Detektieren und Messen von Sakkaden und Pupillenveränderungen mit einer horizontalen Zeilenkamera, einer vertikalen Zeilenkamera und einer Verarbeitungseinheit, die anhand der Signale der Zeilenkameras eine Bewegung des Auges identifiziert und verfolgt, bekannt. Aus US 2008/084539 A1 ist ein Blickverfolgungssystem bekannt, das ein bereitgestelltes Testbild mit einer gemessenen Lichtverteilung, die aus dem Testbild hervorgeht und an Objekten in der Umgebung des Testbilds reflektiert wird, vergleicht.

Der Erfindung liegt die Aufgabe zugrunde, ophthalmologische Geräte der eingangs genannten Art und Verfahren zur Kontrolle einer Fixation eines Auges zu verbessern, so dass die Fixation mit geringem Aufwand, kurzer Reaktionszeit und hoher Genauigkeit überwacht werden kann.

Die Aufgabe wird gelöst durch ein ophthalmologisches Gerät, welches die in Anspruch 1 angegebenen Merkmale aufweist, und durch ein Verfahren, welches die in Anspruch 11 angegebenen Merkmale aufweist.

Vorteilhafte Ausgestaltungen der Erfindung sind in den Unteransprüchen angegeben.

Die Fixationskontrolle an einem Auge gelingt mit geringem Aufwand, kurzer Reaktionszeit und hoher Genauigkeit durch spektroskopische Detektion der Fixation. Aufwendige Polarisationsmessungen sind nicht erforderlich.

Erfindungsgemäß gelingt die spektroskopische Detektion durch mindestens eine Messlichtquelle zur Emission mindestens zweier Wellenlängen, bei denen die Fovea eine andere Reflektivität aufweist als die übrige Retina, eine Optik zur Abbildung der Messlichtquelle auf zumindest einen Teil des Augenhintergrunds und mindestens einen Detektor zum separaten Erfassen der Intensitäten der zwei Wellenlängen in Form eines jeweiligen Detektorsignals nach einer Reflexion an dem Augenhintergrund. Die Messlichtquelle kann dabei mit der Fixierlichtquelle identisch sein oder getrennt von dieser angeordnet werden. Die Messlichtquelle selbst kann in diesem Fall wiederum aus zwei oder mehr Einzellichtquellen bestehen. Zweckmäßigerweise werden die Lichtstrahlen mehrerer Lichtquellen über Strahlvereiniger zusammengeführt. Die beiden Mess-Wellenlängen können in nicht sichtbaren Teil des Spektrums liegen, beispielsweise im Infrarot. Lediglich die Fixierlichtquelle muss zwangsläufig Licht mit einer sichtbaren Wellenlänge (nachfolgend auch als Fixier-Wellenlänge bezeichnet) emittieren. Es ist aber möglich, bei (zumindest teilweiser) Identität von Fixier- und Messlichtquelle eine oder mehrere sichtbare Wellenlängen der Fixierlichtquelle als Mess-Wellenlängen zu verwenden. Beispielsweise kann die Fixierlichtquelle die erste Mess-Wellenlänge und die Messlichtquelle die zweite Mess-Wellenlänge emittieren oder die Messlichtquelle emittiert neben den Mess-Wellenlängen auch die Fixier-Wellenlänge, sei es als eine der Mess-Wellenlängen oder zusätzlich zu diesen. Die Fixierlichtquelle ist vorzugsweise zumindest näherungsweise punktförmig, auch die Messlichtquelle ist vorzugsweise zumindest näherungsweise punktförmig. Die Messlichtquelle wird vorzugsweise punktförmig in den Augenhintergrund abgebildet, bei Identität mit der Fixierlichtquelle ist diese Bedingung zwangsläufig erfüllt. Der Detektor nimmt dann zweckmäßigerweise die Intensitäten der dem Zielort der punktfömigen Abbildung auf der Retina reflektierten Mess-Wellenlängen auf.

Im Zentrum der Retina liegt der sogenannte Gelbe Fleck (Macula lutea) mit etwa 3 mm Durchmesser. In seiner Mitte befindet sich die Fovea centralis, eine Vertiefung mit einem Durchmesser von etwa 1,5 mm. Ein fixiertes Objekt wird in die Fovea abgebildet. Dort ist die räumliche Auflösung des Sehvermögens am höchsten. Im Zentrum der Fovea liegt schließlich die Foveola mit etwa 0,35 mm Durchmesser. Die visuelle Wahrnehmung erfolgt im Bereich der Fovea weitgehend und im Bereich der Foveola ausschließlich über Zapfenzellen. In den restlichen Bereichen des Gelben Flecks ist die Dichte von Stäbchen und Zapfen etwa gleich groß, während die Dichte der Zapfen im Rest der Retina mit zunehmender Entfernung vom Gelben Fleck abnimmt. Die Erfindung beruht auf der Erkenntnis, dass die Fovea (und insbesondere die Foveola) eine andere spektrale Reflektivität als die umgebende Retina (einschließlich der übrigen Fläche der Macula lutea) aufweist. Der Ort einer Reflexion kann daher prinzipiell identifiziert werden, indem der Reflexionsgrad bei einer geeigneten Wellenlänge durch Vergleich der eingestrahlten und der reflektierten Intensität ermittelt und mit einem zu erwartenden Wert verglichen wird. Eine Wellenlänge ist zu diesem Zweck geeignet, wenn die Fovea/Foveola bei dieser Wellenlänge eine andere Reflektivität aufweist als die übrige Retina. Aufwendige Polarisationsmessungen sind nicht erforderlich.

Erfindungsgemäß ist vorgesehen, dass die mindestens zwei verwendeten Mess-Wellenlängen sich in den Verhältnissen ihrer Reflexionsgrade bezüglich der Fovea/Foveola und dem Rest der Retina unterscheiden. Mit anderen Worten, für den Reflexionsgrad $R_{\lambda1,Retina}$ der ersten Wellenlänge bei Reflexion an der Retina, den Reflexionsgrad $R_{\lambda2,Retina}$ der zweiten Wellenlänge bei Reflexion an der Retina, den Reflexionsgrad $R_{\lambda1,Fove(ol)a}$ der ersten Wellenlänge bei Reflexion an der Fovea/Foveola und den Reflexionsgrad $R_{A2,Fove(ol)a}$ der zweiten Wellenlänge bei Reflexion an der Fovea/Foveola muss folgende Bedingung erfüllt sein:

$$R_{\lambda1,Retina} / R_{\lambda2,Retina} \neq R_{\lambda1,Fove(ol)a} / R_{\lambda2,Fove(ol)a}.$$

Die Spezifität der Fixationskontrolle kann durch Verwendung von drei und mehr Mess-Wellenlängen erhöht werden. Diese Wellenlängen müssen die Bedingung der unterschiedlichen Verhältnisse ihrer Reflexionsgrade dann paarweise erfüllen. Prinzipiell ist gemäß der Erfindung auch die Verwendung nur einer einzelnen Mess-Wellenlänge möglich, die bei der Reflexion an der Retina einen anderen Reflexionsgrad aufweist als bei der Reflexion an der Fovea/Foveola:

$$R_{\lambda,Retina} \neq R_{\lambda,Fove(ol)a}.$$

Dann ist jedoch die Separation von Falschlicht aus den detektierten Intensitäten notwendig, wodurch die Fixationskontrolle bedeutend aufwendiger wäre als bei Verwendung mehrerer Wellenlängen.

Die objektive Unterscheidung zwischen fixiertem und unfixiertem Zustand gelingt durch Identifikation des Reflexionsortes (Fovea/Foveola oder Retina) mit geringem Aufwand, in kurzer Zeit und mit hoher Genauigkeit, indem, insbesondere wiederholt, die Intensitätsmessung bei mindestens zwei verschiedenen Mess-Wellenlängen erfolgt und ein Verhältnis der beiden Detektorsignale, die den Intensitätswerten entsprechen, ermittelt und mit einem vorgegebenen Wert oder einem vorgegebenen Wertebereich verglichen wird. In Abhängigkeit des Vergleichsergebnisses wird dann ein Ergebnissignal ausgegeben. Es kann sich um ein elektrisches, akustisches oder elektromagnetisches, insbesondere optisches, Ergebnissignal handeln. Das Ergebnissignal kann kontinuierliche oder nur diskrete (beispielsweise binäre) Werte annehmen. Es kann sich um ein skalares oder mehrdimensionales Ergebnissignal handeln. Beispielsweise kann bei Identifikation eines Übergangs vom fixierten zum unfixierten Zustand ein Warnton ausgegeben werden. Alternativ oder zusätzlich kann das Ergebnissignal eine Entfernung und insbesondere eine Richtung zwischen Fixierlichtquelle und Blickrichtung angeben.

Vorzugsweise sind ein Strahlengang zu dem mindestens einen Detektor und ein Strahlengang zu der mindestens einen Messlichtquelle teilweise identisch. Die Einkopplung kann beispielsweise mittels eines Strahlteilers erfolgen. Eine solche Fixationskontrolleinrichtung kann kompakt gebaut werden und ermöglicht zudem eine hohe Genauigkeit bei der Bestimmung der Reflexionsgrade.

In einer weitergehenden Ausgestaltung ist ein zweidimensionales Feld von Detektoren mit jeweiligen Detektorsignalen und eine entsprechend flächige ausgedehnte Ausbildung der Messlichtquelle zur Emission von ausschließlich unsichtbaren Messwellenlängen vorgesehen, wobei die Messlichtquelle aus Sicht eines Patienten die Fixierlichtquelle zumindest teilweise umgibt. Dadurch kann eine Richtungsangabe für die Fovea/Foveola oder die momentane Blickrichtung ermittelt und insbesondere als Bestandteil des Ergebnissignals ausgegeben werden (ortsaufgelöste Fixationsdetektion). Die Detektoren sind dabei zweckmäßigerweise eineindeutig auf Orte der Retina ausgerichtet, so dass anhand der Reflexionsgrade und anhand der räumlichen Zuordnung ermittelt werden kann, von welchem Ort auf der Retina die Abbildung der Fovea/Foveola erfolgte.

Vorteilhaft ist auch eine alternative Ausgestaltung mit mehreren einzelnen, punktförmig in den Augenhintergrund abbildbaren Messlichtquellen mit jeweils zwei Wellenlängen, die im Falle einer Reflexion an einer Fovea, insbesondere einer Foveola, ein anderes Verhältnis von Reflexionsgraden aufweisen als im Falle einer Reflexion an einer übrigen Retina, und zugeordneten Detektoren, die jeweils die Intensitäten der vom dem jeweiligen Zielort der punktfömigen Abbildungen auf der Retina reflektierten Mess-Wellenlängen aufnehmen. Auch diese Ausgestaltung ermöglicht es, eine Richtungsangabe für die Fovea/Foveola beziehungsweise die momentane Blickrichtung zu ermitteln und insbesondere als Bestandteil des Ergebnissignals auszugeben. Beispielsweise können vier Messlichtquellen und zugeordneten Detektoren in jeweils einem Quadranten des Sehfeldes des Patienten angeordnet sein. In dieser Ausgestaltung wird vorzugsweise am Ort jeder Messlichtquelle eine jeweilige Fixierlichtquelle angeordnet beziehungsweise die Messlichtquelle dient als Fixierlichtquelle, indem eine der Mess-Wellenlängen oder eine zusätzliche Fixier-Wellenlänge im sichtbaren Bereich des Spektrums liegt.

Vorteilhaft sind auch Ausführungsformen, in denen eine einstellbare Projektion der Lichtquellen in unterschiedliche Sehentfernungen möglich ist. Dadurch können Sehfehler des Patienten ausgeglichen werden. Beispielsweise wird für einen Brillenträger durch Abbildung der Lichtquellen in eine geeignete Entfernung die Benutzung des ophthalmologischen Geräts ohne Brille ermöglicht.

Vorteilhaft ist weiterhin eine Mittelung der zur spektroskopischen Auswertung verwendeten Detektorsignale oder mindestens eines daraus ermittelten Verhältnisses über einen vorgegebenen Zeitraum, insbesondere über einen Zeitraum, der länger ist als eine mittlere Verweilzeit des Auges zwischen sakkadischen Bewegungen des Auges, beispielsweise über einen Zeitraum einer Dauer zwischen 20 ms und 100 ms. Diese Glättung dient zur Verbesserung des Signal-Rauschabstandes und damit zur Erhöhung der Genauigkeit der Fixationskontrolle.

Um die Energie der in das Auge eingetragenen Messstrahlung gering genug zu halten, um die Grenzwerte der maximalen Dosis des Auges einzuhalten, dennoch aber genügend Intensität zur Verfügung zu haben, um ein gutes Signal-Rauschverhältnis bei der Detektion zu erzielen, kann die mindestens eine Messlichtquelle zumindest zur Emission einer Mess-Wellenlänge vorzugsweise gepulst betrieben werden. Vorzugsweise können alle Mess-Wellenlängen gepulst emittiert werden.

Zweckmäßigerweise entspricht der Wert oder Wertebereich, der beim Vergleichen verwendet wird, einer Reflexion beider Wellenlängen an der Fovea, insbesondere an der Foveola. Dadurch ergibt sich mit geringem Aufwand ein binäres Ergebnissignal mit der Aussage, ob der Patient momentan die Fixierlichtquelle fixiert oder nicht.

Vorteilhaft ist eine initiale Normierung der Reflexionsverhältnisse an Retina und Fovea, insbesondere Foveola, für das individuelle Auge dadurch, dass ein erster Referenzwert für das Verhältnis der Detektorsignale bei einer Reflexion an der Fovea, insbesondere Foveola, und ein zweiter Referenzwert für das Verhältnis der Detektorsignale bei einer Reflexion an der Retina ermittelt wird. Ermöglicht werden kann diese Normierung beispielsweise durch zwei Fixierlichter, eines auf der Messachse und eines abseits der Messachse, die abwechselnd eingeschaltet werden, so dass das Auge abwechselnd fixiert und nicht fixiert. Der Patient wird dann beispielsweise zunächst gebeten, auf die eine Fixierlichtquelle auf der optischen Achse der spektroskopischen Detektion zu blicken, woraufhin erste Referenzsignale detektiert werden und daraus ein erster Referenzwert für das Reflexionsverhältnis ermittelt wird, und anschließend gebeten wird, auf die andere Fixierlichtquelle abseits der optischen Achse der spektroskopischen Detektion zu blicken, woraufhin zweite Referenzsignale detektiert werden und daraus ein zweiter Referenzwert für das Reflexionsverhältnis ermittelt wird. Der erste Referenzwert entspricht dem zu erkennenden fixierten Zustand des Auges, der zweite Referenzwert entspricht dem unfixierten Zustand. Anhand der beiden Referenzwerte kann ein Kriterium für die Identifikation des Vorliegens der Fixation mit hoher Genauigkeit bestimmt werden. Beispielsweise kann der aus den Referenzwerten ermittelte Mittelwert aus Reflexionsverhältnis an Fovea und Retina oder ein vorgegebener Bruchteil davon als Schwellwert vorgegeben werden, oberhalb dessen auf eine Reflexion an der Fovea (oder der Foveola) geschlossen werden kann.

Alternativ zu der Beleuchtung aus verschiedenen Richtungen mit zwei Fixierlichtquellen, wobei ein Wechsel der Fixierrichtung durch den Patienten erforderlich ist, können zur Normierung die Referenzsignale simultan aus zwei oder mehr Richtungen aufgenommen werden, indem Detektoren mit unterschiedlicher Ausrichtung verwendet werden.

Auch eine sequentielle Aufnahme ist möglich. Alternativ kann für beide Referenzsignale derselbe Detektor verwendet werden, indem verstellbare Strahlablenkvorrichtungen (engl. "scanner") eingesetzt und die Referenzsignale sequentiell von verschiedenen Stellen des Augenhintergrundes aufgenommen werden, beispielsweise einmal von der Fovea/Foveola, einmal von der Retina.

Darüber hinaus kann anhand der Referenzwerte entschieden werden, ob der Kontrast der Reflektionsverhältnisse zwischen Fovea und Retina für eine sichere Detektion der Fixation ausreicht. Beispielsweise kann der Quotient der Referenzwerte gebildet und mit einem vorgegebenen Kontrastschwellwert verglichen werden. Im Falle eines ungenügenden Kontrastes (beispielsweise bei Unterschreiten des Kontrastschwellwertes), beispielsweise aufgrund von Pigmentstörungen der Retina, kann der Bediener darauf hingewiesen werden und muss Maßnahmen ergreifen, um die Fixation manuell zu überprüfen.

Vorteilhafterweise wird in Abhängigkeit des Ergebnissignals, insbesondere durch eine Auswerteeinheit oder eine Steuereinheit, eine Bildaufnahme oder eine Behandlung ausgelöst. Dadurch wird eine zusätzliche menschliche Reaktionszeit vermieden, so dass die Bildaufnahme oder Behandlung beispielsweise im fixierten Zustand beginnt. Der Vorteil der geringen Verzögerung kann erfindungsgemäß allgemein beim Betrieb eines ophthalmologischen Geräts erreicht werden, indem identifiziert wird, dass (mindestens) ein Auge ein Fixierziel fixiert und daraufhin eine Bildaufnahme von dem Auge oder eine Behandlung des Auges ausgelöst und/oder eine Bewegungsverfolgung des Auges eingerastet wird. Entsprechend umfasst die Erfindung auch eine Fixationskontrolleinrichtung für ein ophthalmologisches Gerät mit einer Auswerteeinheit, welche einen Zustand einer Fixation eines Fixierziels durch ein Auge identifiziert und daraufhin eine Bildaufnahme von dem Auge oder eine Behandlung des Auges auslöst und/oder eine Bewegungsverfolgung des Auges einrastet. Eine Bewegungsverfolgung kann auf bekannte Weise beispielsweise durch wiederholte Bildaufnahme der Pupille des Auges erfolgen. Insbesondere kann in allen Fällen die Bildaufnahme beziehungsweise Behandlung dann bei Identifikation eines Verlustes der Fixation automatisch abgebrochen werden. Je nach Art der Bildaufnahme beziehungsweise Behandlung kann sie gegebenenfalls bei Detektion der Wiederherstellung der Fixation fortgesetzt werden.

Indem die ermittelten Werte der Reflexionsverhältnisse graduell interpretiert werden, kann eine Wertung und/oder Wichtung der Messergebnisse und/oder der Bilder des ophthalmologischen Gerätes erfolgen. Dazu nimmt das Gerät eine Serie von Messwerten und/oder Bildern auf und ermittelt währenddessen auf die erfindungsgemäße Weise für jeden Messwerte und/oder jedes Bild das momentane Reflexionsverhältnis als Wert des momentanen Grades der Fixation und ordnet den Messwerten und/oder Bildern den jeweiligen Wert des Grades der Fixation zu. Anhand der jeweiligen Werte des Grades der Fixation kann dann ein gewichteter Mittelwert der von dem Gerät aufgenommenen Messwerte und/oder Bilder ermittelt werden. Das kann beispielsweise das Ausschließen bestimmter Messwerte und/oder Bilder der Serie bei zu geringem Grad der Fixation durch die Gewichtung Null bedeuten. Im Extremfall wird der einzelne Messwert beziehungsweise das einzelne Bild selektiert und angezeigt, das den höchsten Grad der Fixation aus der aufgenommenen Serie aufweist.

In einer bevorzugten Ausführungsform erfolgt eine Modulation der Fixierlichtquelle in Abhängigkeit des Ergebnissignals. Dies kann auch als Rückkopplung des Ergebnissignals auf die Intensität des Fixierlichts bezeichnet werden. Beispielsweise beginnt die Fixierlichtquelle zu blinken, wenn detektiert wird, dass der Patient sie nicht mehr fixiert, dass also das Fixierlicht nicht mehr an der Fovea reflektiert wird. Durch die visuelle Stimulation wird der Patient unwillkürlich dazu gebracht, wieder auf das Fixierlicht zu blicken.

Vorteilhafterweise erfolgt eine Intensitätsmodulation der mindestens einen Messlichtquelle. Dadurch wird zur Messung der Intensitäten beider (bzw. mehrerer) Mess-Wellenlängen nur ein Detektor benötigt. Dies gelingt entweder durch eine Modulation mit für die Wellenlängen unterschiedlichen Frequenzen und Erfassung der reflektierten Mess-Wellenlängen in einem gemeinsamen Detektor, wobei anschließend beispielsweise durch die sogenannte Lock-in-Technik eine elektronische Trennung in die jeweiligen Detektorsignale erfolgt, oder durch eine Modulation mit für die Wellenlängen identischer Frequenz bei versetzten Phasen und phasensensitiver Erfassung der Wellenlängen in einem gemeinsamen Detektor.

Besonders bevorzugt ist eine Ausgestaltung mit konfokaler Detektion in dem mindestens einen Detektor. Die konfokale Detektion an einem zum betrachteten Reflexionsort konjugierten Punkt ermöglicht die Messung der Reflexionsgrade mit hoher räumlicher Auflösung.

In einer Weiterbildung der Erfindung erfolgt die Aufnahme eines Bildes einer Iris zur Bestimmung einer relativen Position einer Pupille, insbesondere einer Pupillenmitte, zu einem Vertex einer Cornea bezüglich einer optischen Bezugsachse mittels einer Kamera. Der momentane Vertex V ist derjenige Punkt der Cornea, der der Kamera längs der optischen Bezugsachse am nächsten liegt. Mit anderen Worten, der Vertex bezüglich der optischen Achse ist derjenige Punkt der Oberfläche, dessen Orthogonalprojektion auf die optische Achse die kleinste Entfernung zur Eintrittsoptik der Kamera aufweist. Alternativ kann er als Gipfelpunkt (höchster Punkt) in Blickrichtung der optischen Achse der Kamera oder als lokales Maximum oder Extremum bezüglich der optischen Achse der Kamera definiert werden. Zweckmäßigerweise wird der Ort des Vertex anhand eines Purkinje-Reflexes an der Cornea ermittelt. Dies erfolgt, wenn mittels der erfindungsgemäßen Fixationskontrolleinrichtung die Fixation der Fixierlichtquelle identifiziert wurde, entweder durch die Fixationskontrolleinrichtung selbst oder durch das übergeordnete ophthalmologische Gerät. Dadurch kann die ermittelte Pupillen-Vertex-Relation der zur identifizierten Fixation gehörigen Blickrichtung zugeordnet werden. Da sich die Lagerelation von Pupille und Vertex mit der Blickrichtung ändert, kann das im als Fixation identifizierten Zustand aufgenommene Bild oder die daraus ermittelte Pupillen-Vertex-Relation als Referenz für eine korrekte Fixation verwendet werden. Bei einigen Diagnose- und/oder Therapieverfahren wird die momentane Pupillen-Vertex-Relation zu anderen Zwecken ermittelt und kann so auch zur Fixationskontrolle genutzt werden. Insbesondere in Ausgestaltungen mit ortsaufgelöster Fixationsdetektion können mehrere Pupillen-Vertex-Relation (insbesondere in Form von Irisbildern) für unterschiedliche Fixationsrichtungen ermittelt und gespeichert werden. Bei genügender Anzahl und Dichte solcher Stützpunkte kann beispielsweise durch Interpolation eine Erkennung von Augenbewegungen durchgeführt werden. Die Ermittlung einer oder mehrerer Pupillen-Vertex-Relationen kann vorteilhafterweise unabhängig von einer späteren Nutzung durchgeführt werden, insbesondere präoperativ.

Vorteilhafterweise kann die Fixationskontrolleinrichtung binokular ausgebildet sein, um die Fixation zweier Augen simultan überwachen zu können. Dies kann bei Geräten genutzt werden, die binokulare Augenvermessungen vornehmen. Beispielsweise kann eine beidseitige Fixationsdetektion zur Analyse des stereoskopischen Sehens oder einer Fehlstellung der Augen (Strabismus) dienen. Zu diesem Zweck kann insbesondere die ortsaufgelöste Fixationskontrolle mit Ermittlung der Blickrichtung verwendet werden.

In einer vorteilhaften Ausprägung ist die erfindungsgemäße Fixationskontrolleinrichtung als Modul ausgebildet, so dass herkömmliche Fixierlichtquellen mit geringem Aufwand ersetzt werden können.

Die Erfindung umfasst auch ein ophthalmologisches Gerät mit einer erfindungsgemäßen Fixationskontrolleinrichtung sowie Computerprogramme oder Auswerteeinheiten, die zur Durchführung eines erfindungsgemäßen Verfahrens eingerichtet sind. Ein Computerprogramm kann zu diesem Zweck beispielsweise ein Softwaremodul zum Beleuchten zumindest eines Teils eines Augenhintergrunds mit Licht zweier Wellenlängen, die im Falle einer Reflexion an einer Fovea, insbesondere an einer Foveola, ein anderes Verhältnis von Reflexionsgraden aufweisen als im Falle einer Reflexion an einer Retina, mittels mindestens einer Messlichtquelle, ein Softwaremodul zum separaten Erfassen der Intensitäten der zwei Wellenlängen mittels mindestens eines Detektors als jeweiliges Detektorsignal nach einer Reflexion an einem Augenhintergrund, ein Softwaremodul zum Ermitteln eines Verhältnisses der beiden Detektorsignale, ein Softwaremodul zum Vergleichen des Verhältnisses mit einem vorgegebenen Wert oder einem vorgegebenen Wertebereich und ein Softwaremodul zum Ausgeben eines Ergebnissignals in Abhängigkeit des Vergleichsergebnisses umfassen. Dabei kann ein einzelnes Softwaremodul alle obigen Aufgaben erfüllen. Alternativ kann zumindest ein einzelnes Softwaremodul einige der obigen Aufgaben erfüllen. Es kann sich aber auch um fünf oder mehr verschiedene Softwaremodule handeln.

Nachfolgend wird die Erfindung anhand von Ausführungsbeispielen näher erläutert.

In den Zeichnungen zeigen:

Fig. 1 ein ophthalmologisches Gerät mit einer Fixationskontrolleinrichtung,

Fig. 2 eine weitere Fixationskontrolleinrichtung,

Fig. 3 ein Schaltschema für die Messlichtquellen dieser Fixationskontrolleinrichtung,

Fig. 4 eine dritte Fixationskontrolleinrichtung,

Fig. 5 eine vierte Fixationskontrolleinrichtung,

Fig. 6 eine fünfte Fixationskontrolleinrichtung,

Fig. 7 ein weiteres ophthalmologisches Gerät,

Fig. 8 eine sechste Fixationskontrolleinrichtung,

Fig. 9 eine siebte Fixationskontrolleinrichtung und

Fig. 10 eine achte Fixationskontrolleinrichtung.

In allen Zeichnungen tragen übereinstimmende Teile gleiche Bezugszeichen.

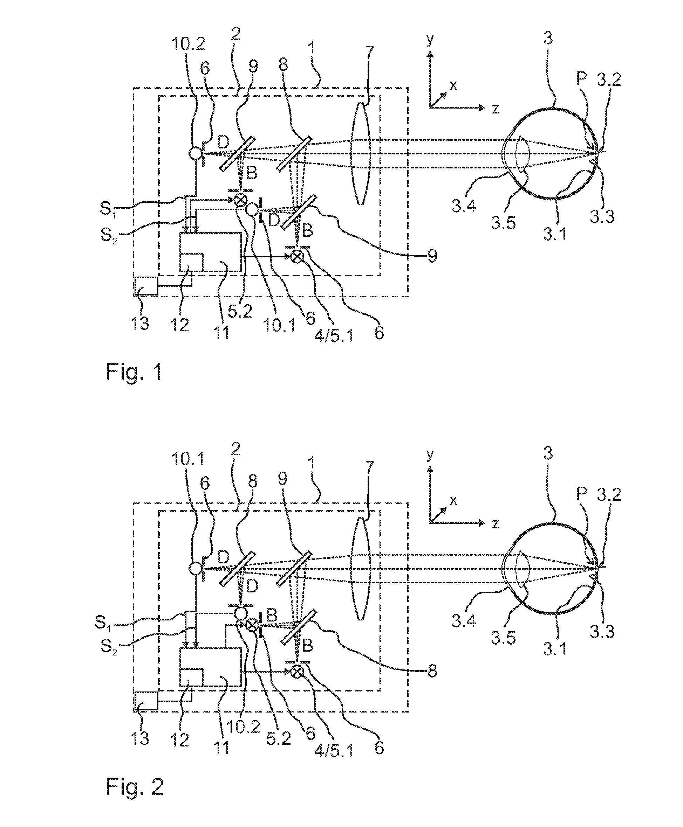

**Fig. 1** zeigt ein ophthalmologisches Gerät 1, beispielsweise einen refraktiven Laser, mit einer Fixationskontrolleinrichtung 2 zur Überwachung der Fixation eines Auges 3 in einer schematischen Darstellung. Die Fixationskontrolleinrichtung 2 umfasst eine beispielsweise näherungsweise punktförmige Fixierlichtquelle 4, die gleichzeitig als erste Messlichtquelle 5.1 dient, eine zweite Messlichtquelle 5.2, den Lichtquellen 4 und 5 zugeordnete Lochblenden 6, eine abbildende Optik 7, einen dichroitischen Farbteiler 8, zwei farbneutrale Strahlteiler 9, zwei Photodetektoren 10.1 und 10.2, denen ebenfalls jeweils eine Lochblende 6 zugeordnet ist, und eine Steuer- und Auswerteeinheit 11, die über eine Ausgabeschnittstelle 12 verfügt. Die Ausgabeschnittstelle 12 ist beispielsweise mit einem Piezosummer als Ausgabeeinheit 13 verbunden. Die Fixierlichtquelle 4 emittiert beispielsweise ausschließlich sichtbares Licht einer Fixier-Wellenlänge $\lambda_F$, die auch als erste Mess-Wellenlänge $\lambda_1$ verwendet wird: $\lambda_F = \lambda_1 = 590$ nm. Die zweite Messlichtquelle 5.2 emittiert beispielsweise ausschließlich unsichtbares Infrarotlicht einer zweiten Wellenlänge $\lambda_2 = 1\,050$ nm. Der Farbteiler 8 ist so ausgelegt, dass ihn die erste Mess-Wellenlänge $\lambda_1$ durchdringt, während die zweite Mess-Wellenlänge $\lambda_2$ an ihm gespiegelt wird. Die beiden Lichtquellen 4/5.1 und 5.2 werden beispielsweise elektronisch auf ein vorgegebenes Verhältnis der Lichtintensitäten der beiden Mess-Wellenlängen $\lambda_1$, $\lambda_2$ geregelt. Alternativ kann die relative Intensität anhand einer Referenzmessung ermittelt werden.

Werden die Lichtquellen 4 und 5 von der Steuer- und Auswerteeinheit 11 eingeschaltet, so bilden die Lochblenden 6 und die Optik 7 die Lichtquellen 4, 5 über die Strahlteiler 8 und 9 jeweils punktförmig auf einen gemeinsamen Punkt P auf dem Augenhintergrund 3.1 ab, wenn der Patient sein Auge 3 auf die Fixier-Lichtquelle 4 fixiert. Dieser Punkt P kann je nach Stellung des Auges 3 auf der Fovea 3.2 (insbesondere der Foveola) oder im Bereich der übrigen Retina 3.3 liegen. Die Lochblenden 6 vor den Detektoren 10 sind in einer jeweiligen, mit dem beleuchteten Punkt P konjugierten Ebene angeordnet, so dass die Detektion in den Detektoren 10 in diesem Ausführungsbeispiel konfokal erfolgt. Durch die Kopplung der Beleuchtungsstrahlengänge B und der Detektionsstrahlengänge D über die Strahlteiler 8 und 9 erscheinen vom Standpunkt des Auges 3 alle Lichtquellen 4, 5 optisch am selben Ort. In Verbindung mit der konfokalen Detektion nehmen die Detektoren 10 entsprechend ausschließlich Licht aus demselben Punkt P auf und erzeugen daraus elektrische Signale $S_1$, $S_2$, die die aufgenommenen Lichtintensitäten der beiden Mess-Wellenlängen $\lambda_1$, $\lambda_2$ separat repräsentieren.

Die verwendeten Fixier- und Messwellenlängen $\lambda_1$, $\lambda_2$ erfüllen die Bedingung einer zwischen Fovea 3.2 und dem Bereich der übrigen Retina 3.3 unterschiedlich starken Reflexion und insbesondere die Bedingung unterschiedlicher Verhältnisse ihrer Reflexionsgrade bei Reflexionen an Fovea 3.2 beziehungsweise Retina 3.3. Sie unterscheiden sich also in ihrer relativen spektralen Reflektivität bezüglich Fovea 3.2 und Retina 3.3:

$$R_{\lambda 1, Retina} / R_{\lambda 2, Retina} \neq R_{\lambda 1, Fove(ol)a} / R_{\lambda 2, Fove(ol)a}.$$

Zur Fixationskontrolle schaltet die Steuer- und Auswerteeinheit 11 die Lichtquellen 4 und 5 permanent ein und wertet die beiden elektrischen Signale $S_1$, $S_2$ aus, indem sie sie digitalisiert und zueinander ins Verhältnis setzt. In alternativen Ausgestaltungen (nicht abgebildet) kann das auch analog-elektrisch erfolgen. Die resultierende Größe $Q = S_1/S_2$ vergleicht die Steuer- und Auswerteeinheit 11 beispielsweise mit einem vorgegebenen Schwellwert, oberhalb dessen aufgrund der unterschiedlichen Reflexionsgrade auf eine Reflexion an der Fovea 3.2 oder der Foveola (nicht abgebildet) geschlossen werden kann. Beispielsweise reflektiert die Fovea 3.2 aufgrund ihrer gelben Pigmentierung die erste Mess-Wellenlänge $\lambda_1$ signifikant stärker als die zweite Mess-Wellenlänge $\lambda_2$. In der Folge ist der relative Reflexionsgrad und damit die messbare Intensität der ersten Mess-Wellenlänge $\lambda_1$ bei einer Reflexion an der Fovea 3.2 größer als bei der Reflexion an der übrigen Retina 3.3. Für die Mess-Wellenlänge $\lambda_2$ gilt beispielsweise ein etwa entgegengesetztes Reflexionsverhalten.

Anhand des Verhältnisses der gemessenen Lichtintensitäten der beiden Mess-Wellenlängen $\lambda_1$, $\lambda_2$ kann also ermittelt werden, ob die gemessene Reflexion an der Fovea 3.2 oder im Bereich der übrigen Retina 3.3 stattfand. Sofern das Auge 3 auf die Fixier-Lichtquelle 4 fixiert ist, liegt das Ziel P der punktförmigen Abbildung der Fixier-Lichtquelle 4 und damit auch der Ort der in den Detektoren 10 gemessenen Reflexion auf der Fovea 3.2. Durch den Vergleich mit dem Schwellwert kann die Steuer- und Auswerteeinheit 11 ermitteln, ob im Zeitpunkt der Messung eine korrekte Fixation vorlag. Da die Messung und der Vergleich mit hoher Geschwindigkeit durchgeführt werden können (eine Bildverarbeitung ist nicht notwendig), kann das Ergebnissignal mit kurzer Reaktionszeit ausgegeben werden. Selbstverständlich kann der Quotient der elektrischen Signale je nach verwendeten Wellenlängen invers gebildet werden: $Q = S_2/S_1$. Abhängig von der Bildung des Quotienten kann die Fixation anhand eines Über- oder Unterschreitens des vorgegebenen Schwellwerts identifiziert werden.

Sofern die resultierende Größe kleiner als der Schwellwert ist, gibt die Steuer- und Auswerteeinheit 11 im vorliegenden Ausführungsbeispiel als digitales elektrisches Ergebnissignal einen 1-Pegel über die Schnittstelle 12 aus. Die Ausgabeeinheit 13 erzeugt daraufhin einen Warnton, so dass der Behandler und der Patient über den Verlust der Fixation informiert werden. Ist die resultierende Größe größer oder gleich dem Schwellwert, wird ein O-Pegel als Ergebnissignal ausgegeben. Dadurch unterbleibt der Warnton oder wird ausgeschaltet, sofern er zuvor eingeschaltet war.

In alternativen Ausgestaltungen (nicht abgebildet) kann das Ergebnissignal alternativ oder zusätzlich zu der beschriebenen Ausgabeeinheit 13 von einer Steuereinheit (nicht abgebildet) des ophthalmologischen Geräts 1 verwendet werden, um eine Untersuchung und/oder eine Behandlung abzubrechen, sobald das Ergebnissignal einen Verlust der Fixation anzeigt. Zusätzlich oder alternativ ist es auch möglich, eine Untersuchung und/oder Behandlung automatisch auszulösen, wenn anhand des Ergebnissignals feststeht, dass die Fixier-Lichtquelle 4 von dem Auge 3 korrekt fixiert ist.

Um zu vermeiden, dass eine zufällige Augenbewegung als Fixation identifiziert wird, kann die zusätzliche Bedingung aufgestellt werden, dass die identifizierte Fixation für eine Mindestdauer bestehen muss, bevor eine Folge ausgelöst wird. Die Prüfung einer Mindestdauer kann in der Fixationskontrolleinrichtung 2 selbst oder nachgeordnet im ophthalmologischen Gerät 1 erfolgen. Beispielsweise kann die Steuer- und Auswerteeinheit 11 bei erstmaliger Ermittlung eines Vergleichsergebnisses, das eine Fixation anzeigt, einen Zeitkontrollzähler initialisieren. Nur wenn die Fixation bis zum Ablauf der Mindestdauer von beispielsweise einer Sekunde ununterbrochen aufrechterhalten wurde, wird ein die Fixation anzeigendes Ergebnissignal ausgegeben.

Anstelle dichroitischer Farbteiler 8 können zur Selektion der Mess-Wellenlängen Neutralteiler mit Farbfiltern, Gittern und/oder Prismen kombiniert werden (nicht abgebildet). In weiteren Ausgestaltungen (nicht abgebildet) können mehr als zwei Mess-Wellenlängen verwendet werden. Es werden dann drei oder mehr elektrische Signale S gemessen und beispielsweise paarweise ins Verhältnis gesetzt. Zur Identifikation einer korrekten Fixation wird dann beispielsweise für jeden auf diese Weise ermittelten Quotienten ein separater Vergleich mit einem jeweiligen Schwellwert durchgeführt. Im Ergebnissignal wird dann nur eine korrekte Fixation angezeigt, wenn jede der jeweils vorgegebenen Bedingungen (Über- oder Unterschreiten) erfüllt ist.

Auf Lochblenden 6 vor den Detektoren 10 kann verzichtet werden (nicht abgebildet), wenn die Detektoren 10 eine geringe Apertur aufweisen. Die Detektoren 10 werden dann anstelle der Lochblenden 6 in einer konfokalen Ebene angeordnet. Beispielsweise kann ein einzelner Pixel eines konfokal angeordneten, ortsauflösenden Sensors ohne Lochblende 6 als konfokaler Detektor 10 verwendet werden.

In weiteren Ausführungsformen (nicht abgebildet) kann die abbildende Optik 7 mittels einer Motorsteuerung so ausgebildet sein, dass sie auf unterschiedliche Sehentfernungen des Auges 3 einstellbar ist. Zu diesem Zweck ist die Steuer- und Auswerteeinheit 11 in solchen Ausführungsformen zur Beeinflussung der Motorsteuerung mit dieser verbunden.

Fig. 2 zeigt eine alternative Ausführungsform, bei der die Detektoren 10 an einem ersten Farbteiler 8 und die Lichtquellen 4, 5 an einem zweiten Farbteiler 8 zusammengezogen sind. Der Beleuchtungsstrahlengang B und der Detektionsstrahlengang D werden an einem Neutralteiler 9 gekoppelt. Im übrigen wird auf die Beschreibung zu Fig. 1 verwiesen.

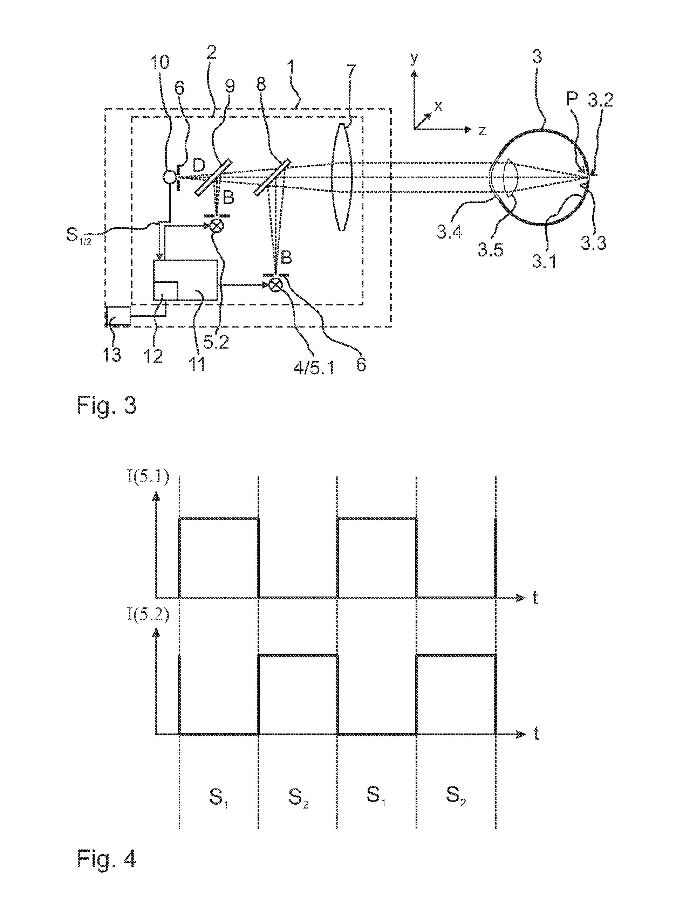

In Fig. 3 ist eine Fixationskontrolleinrichtung 2 gezeigt, die weitgehend mit der Ausführungsform gemäß Fig. 1 übereinstimmt. Im Unterschied zu dieser ist jedoch nur ein einzelner Detektor 10 zur Lichtaufnahme für beide Mess-Wellenlängen $\lambda_1$, $\lambda_2$ vorgesehen. Der Detektor 10 gibt nur ein einzelnes elektrisches Signal $S_{1/2}$ ab. Zur Zweck der Trennung werden die beiden Messlichtquellen 5.1, 5.2 und damit auch die Fixierlichtquelle 4 intensitätsmoduliert. In der Folge blinken sie hochfrequent. Dies kann für die beiden Messlichtquellen 5.1 und 5.2 mit gleichen oder mit unterschiedlichen Frequenzen erfolgen. Im erstgenannten Fall erfolgt die Modulation mit versetzter Phase, was in Fig. 4 schematisch illustriert ist. Der Detektor 10 nimmt hier phasensensitiv entweder die eine oder die andere Mess-Wellenlänge $\lambda_1$, $\lambda_2$ auf, so dass das elektrische Signal $S_{1/2}$ von der Steuer- und Auswerteeinheit 11 blockweise als erstes Signal $S_1$ oder als zweites Signal $S_2$ interpretiert wird. Im zweitgenannten Modulationsfall mit unterschiedlichen Modulationsfrequenzen ist es nötig, die detektierten Intensitäten der beiden Mess-Wellenlängen $\lambda_1$, $\lambda_2$ elektronisch zu separieren, beispielsweise mittels Lock-In-Technik, bei der das Modulationssignal als Referenz verwendet wird. Zweckmäßigerweise liegt zumindest die Modulationsfrequenz der sichtbaren Fixier- und ersten Messlichtquelle 4/5.1 bei beiden Modulationsarten so hoch, dass das Auge 3 keine Modulation wahrnimmt, also beispielsweise bei 50 Hz, 100 Hz oder 1 kHz. Insbesondere die Modulation mit unterschiedlichen Frequenzen kann mit einer statischen Farbselektion mittels Strahlteilern/Filtern/Gittern/Prismen kombiniert werden.

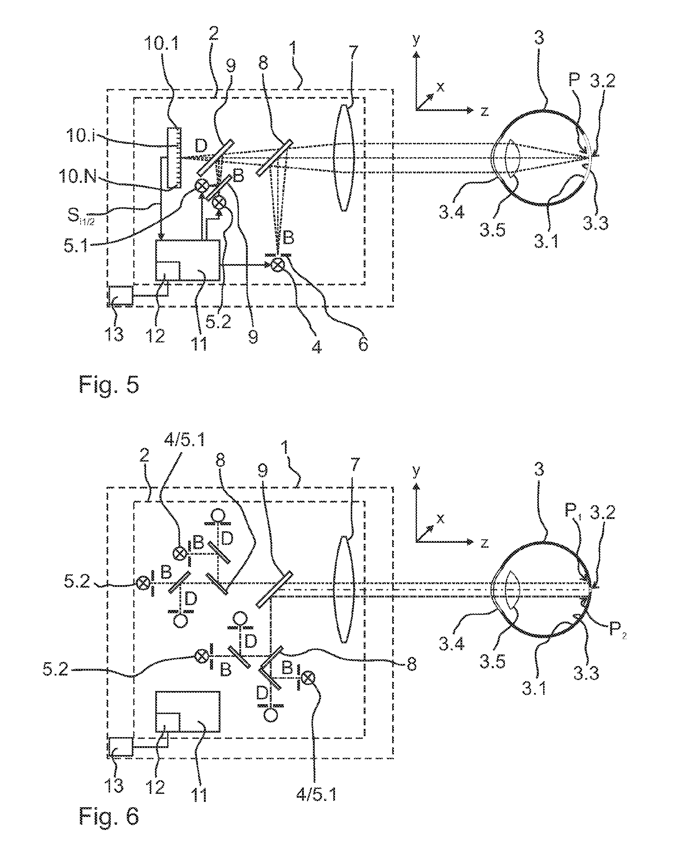

Fig. 5 zeigt eine Fixationskontrolleinrichtung 2, die weitgehend mit der Ausführungsform gemäß Fig. 2 übereinstimmt. Im Unterschied zu dieser sind jedoch ein zweidimensionales Feld von Einzeldetektoren 10.i (i=1...N, z. B. N=256) und entsprechend flächig in den Augenhintergrund 3.1 abgebildete Messlichtquellen 5.1 und 5.2 neben einer separaten, punktförmig in den Augenhintergrund 3.1 abgebildeten Fixierlichtquelle 4 vorgesehen. Die beiden Lichtquellen 5.1 und 5.2 werden beispielsweise elektronisch auf ein vorgegebenes Verhältnis der Lichtintensitäten der beiden Mess-Wellenlängen $\lambda_1$, $\lambda_2$ geregelt. Alternativ kann die relative Intensität anhand einer Referenzmessung ermittelt werden. Aus der Sicht des Auges 3 umgeben die flächigen Messlichtquellen 5.1 und 5.2 (im Augenhintergrund 3.1 weiß dargestellt) die punktförmige Fixierlichtquelle 4 vollständig. Aus der Sicht der Fixationskontrolleinrichtung 2 blicken die

Einzeldetektoren 10.i auf unterschiedliche Orte des Augenhintergrunds 3.1 und erlauben somit, die Lage der Fovea 3.2 (oder insbesondere der Foveola) anhand der Intensitäten der unterschiedlich reflektierten Mess-Wellenlängen $\lambda_1$, $\lambda_2$ zu ermitteln. Die Messlichtquellen 5.1 und 5.2 werden zu diesem Zweck intensitätsmoduliert betrieben wie oben zu Fig. 3 beschrieben. Die Fixierlichtquelle 4 leuchtet permanent. Indem für jeden Einzeldetektor 10.i separate elektrische Signale $S_{i,1/2}$ gemessen und detektorweise ins Verhältnis gesetzt werden, kann für jeden Einzeldetektor 10.i identifiziert werden, ob die Fovea 3.2 auf ihn abgebildet wurde. Aus der relativen Lage des Abbilds der Fovea 3.2 im Detektorfeld kann die momentane Blickrichtung des Auges 3 ermittelt und über die Ausgabeschnittstelle 12 ausgegeben werden.

Zweckmäßigerweise sind einem solchen Fall zur Weiterverarbeitung Auswerteeinheiten (nicht abgebildet) des ophthalmologischen Geräts 1 mit der Schnittstelle 12 verbunden. Wenn das Auge 3 die Fixierlichtquelle 4 nur unscharf fixiert, wird die Fovea 3.2 beispielsweise als verwaschener Fleck auf den betreffenden Detektor 10.i abgebildet. Als Ort des Bildes der Fovea 3.2 wird dann beispielsweise ein Zentrum des Flecks ermittelt.

Auch in **Fig. 6** ist eine Fixationskontrolleinrichtung 2 gezeigt, die weitgehend mit der Ausführungsform gemäß Fig. 1 übereinstimmt, so dass insoweit auf deren Beschreibung verwiesen wird. Im Unterschied zu dieser sind jedoch mehrere unabhängige Beleuchtungs- und Detektionsstrahlengänge B, D mit jeweils einer eigenen Fixierlichtquelle 4 vorgesehen. Der Übersichtlichkeit halber sind nur zwei Beleuchtungs- und Detektionsstrahlengänge B, D abgebildet. In allen Strahlengängen B, D kann dasselbe Paar (Tripel, Quadrupel usw. bei mehr als zwei Wellenlängen) von Mess-Wellenlängen $\lambda_1$, $\lambda_2$ verwendet werden. Diese Anordnung ermöglicht ebenfalls die ortsaufgelöste Fixationsdetektion, da identifiziert werden kann, welche der Fixierlichtquellen 4 von dem Auge 3 fixiert wird, sofern überhaupt eine Fixation vorliegt.

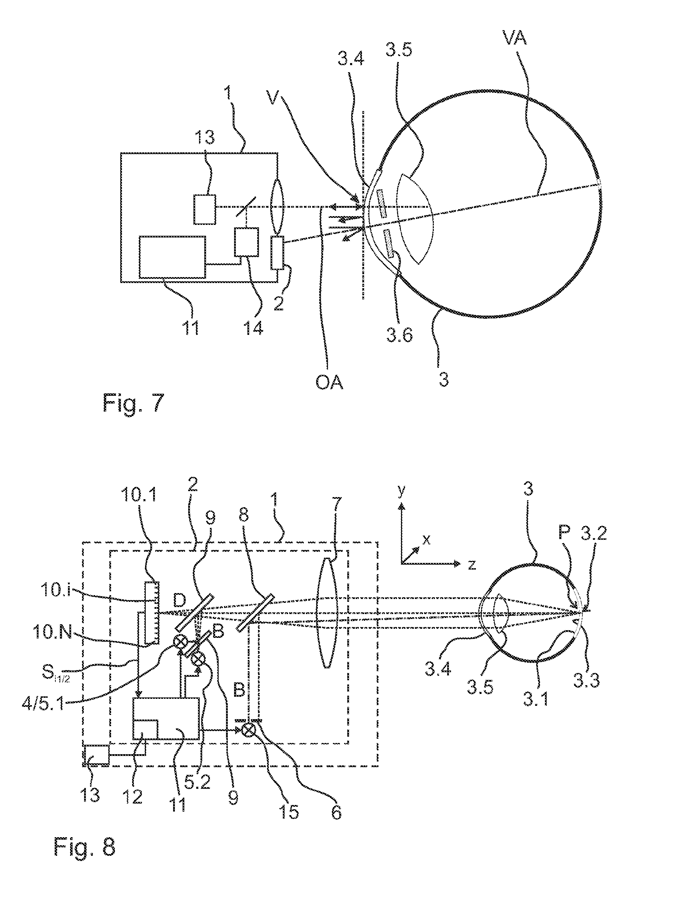

Schließlich illustriert **Fig. 7** schematisch ein ophthalmologisches Gerät 1, beispielsweise ein Femtosekunden-Laser zur chirurgischen Behandlung, das zusätzlich zu einer Fixationskontrolleinrichtung 2 mit einer Lichtquelle 13 zur kollimierten Beleuchtung der Cornea 3.4 und mit einer Kamera 14 zur Aufnahme eines Bildes insbesondere der Pupille 3.6 ausgerüstet ist. Die Steuer- und Auswerteeinheit 11 kann eine Lage des Vertex V der Cornea 3.4 bezüglich der optischen Achse OA der Kamera 14 (identisch mit der optischen Achse des nicht abgebildeten Lasers) im Koordinatensystem des Lasers (typischerweise kartesische Koordinaten x/y/z mit z in Richtung der optischen Achse des Lasers) messen, indem sie mittels der Lichtquelle 13 und der Kamera 14 und in bekannter Weise einen Purkinje-Reflex erzeugt und anhanddessen den momentanen Vertex lokalisiert. Der Fixierpunkt für den Patienten (hier nicht abgebildete Fixierlichtquelle der Fixationskontrolleinrichtung 2) liegt abseits der optischen Achse OA. Das kollimierte, parallel zu dieser Achse auf die Cornea 3.4 fallende Licht wird nur von dem höchsten Punkt der Cornea 3.4, dem Vertex V, zur Kamera 14 reflektiert. Reflexionen von anderen Stellen erreichen die Kamera 14 nicht (durch Pfeile angedeutet). Wenn die Steuer- und Auswerteeinheit mittels der Fixationskontrolleinrichtung 2 identifiziert, dass das Auge 3 die Fixierlichtquelle (hier nicht abgebildet) der Fixationskontrolleinrichtung 2 fixiert, wird zudem ein Bild beispielsweise der Pupille 3.6 aufgenommen. Die relative Lage des momentanen Vertex V (Fixationsvertex) zu einem augenfesten Bezugspunkt, beispielsweise dem Schwerpunkt oder Mittelpunkt der Pupille 3.6 oder dem Limbus, wird dann in Form eines zweidimensionalen Vektors anhand des aufgenommenen Bildes ermittelt und gespeichert/ausgegeben oder in Form des gesamten aufgenommenen Bildes als Referenz für den fixierten Zustand gespeichert/ausgegeben.

In allen Ausführungsformen können anstelle von punktförmigen Fixierlichtquellen andere Ausgestaltungen verwendet werden, die einen anzuvisierenden Punkt eindeutig definieren, beispielsweise kreuzförmige Fixierlichtquellen.

In **Fig. 8** ist eine Ausführungsform einer Fixationskontrolleinrichtung 2 mit einer zusätzlichen Fixierlichtquelle 15 dargestellt, die abseits der optischen Achse des Detektors 10 angeordnet ist. Diese Ausführungsform ermöglicht die individuelle Normierung auf das Auge 3. Wenn der Blick VA des Auges 3 auf die erste Fixierlichtquelle 4 gerichtet ist, können mittels des Detektors 10 erste Referenzsignale für den fixierten Zustand des Auges 3 ermittelt werden, da die Mess-Wellenlängen an der Fovea 3.2 reflektiert werden. Wenn der Blick VA des Auges 3 auf die zusätzliche Fixierlichtquelle 15 gerichtet ist, können mittels des Detektors 10 zweite Referenzsignale für den nicht fixierten Zustand des Auges 3 ermittelt werden, da die Mess-Wellenlängen abseits der Fovea 3.2 reflektiert werden. Aus den ersten Referenzsignalen kann ein erster Referenzwert und aus den zweiten Referenzsignalen kann ein zweiter Referenzwert ermittelt werden. Der arithmetische Mittelwert der beiden Referenzwerte kann beispielsweise als Schwellwert zur Identifikation der Fixation vorgegeben werden.

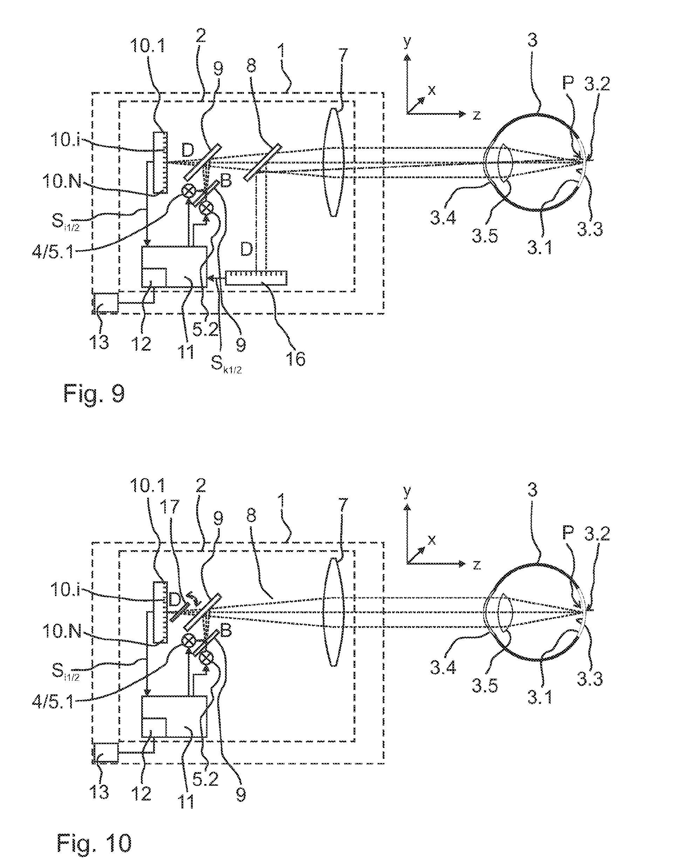

Eine abgewandelte Ausführungsform mit Normierungsmöglichkeit zeigt **Fig. 9.** Hier nimmt ein zweiter Detektor 16 simultan mit dem ersten Detektor 10 Licht auf, dass an einer anderen Stelle reflektiert wurde als das Licht, dass der erste Detektor 10 aufnimmt. Wenn der Blick VA des Auges 3 auf die Fixierlichtquelle 4 gerichtet ist, können mittels des ersten Detektors 10 erste Referenzsignale für den fixierten Zustand des Auges 3 ermittelt werden, da die aufgenommenen Mess-Wellenlängen an der Fovea 3.2 reflektiert wurden, und mittels des zweiten Detektors 16 zweite Referenzsignale für den nicht fixierten Zustand des Auges 3 ermittelt werden, da die aufgenommenen Mess-Wellenlängen abseits der Fovea 3.2 reflektiert wurden.

**Fig. 10** zeigt eine weitere Ausführungsform mit Normierungsmöglichkeit, in der im Detektionsstrahlengang eine einstellbare Strahlablenkvorrichtung 17 angeordnet ist. Durch Verstellen der Strahlablenkvorrichtung 17 kann der Detektionsstrahlengang D abgelenkt werden, so dass Detektorsignale von unterschiedlichen Stellen des Augenhintergrunds aufgenommen werden können. Wenn der Detektionsstrahlengang D auf die Fovea 3.2 gerichtet ist, können mittels des Detektors 10 erste Referenzsignale für den fixierten Zustand des Auges 3 ermittelt werden, da die aufgenommenen Mess-Wellenlängen an der Fovea 3.2 reflektiert wurden. Wenn der Detektionsstrahlengang D auf eine Stelle auf der übrigen Retina 3.3 gerichtet ist, können mittels des Detektors 10 zweite Referenzsignale für den nicht fixierten Zustand des Auges 3 ermittelt werden, da die aufgenommenen Mess-Wellenlängen an der Retina 3.3 reflektiert wurden.

Bezugszeichenliste

| | |
|---|---|
| 1 | Ophthalmologisches Gerät |
| 2 | Fixationskontrolleinrichtung |
| 3 | Auge |
| 3.1 | Augenhintergrund |
| 3.2 | Fovea |
| 3.3 | Retina |
| 3.4 | Cornea |
| 3.5 | Augenlinse |
| 3.6 | Pupille |
| 4 | Fixierlichtquelle |
| 5 | Messlichtquellen |
| 5.1 | Erste Messlichtquelle |
| 5.2 | zweite Messlichtquelle |
| 6 | Lochblende |
| 7 | Optik |
| 8 | Farbteiler |
| 9 | Neutralteiler |
| 10 | Detektor |
| 10.1 | Erster Detektor |
| 10.2 | Zweiter Detektor |
| 11 | Steuer- und Auswerteeinheit |
| 12 | Ausgabeschnittstelle |
| 13 | Lichtquelle |
| 14 | Kamera |
| 15 | Zusätzliche Fixierlichtquelle abseits der optischen Achse |
| 16 | Zweiter Detektor |
| 17 | Einstellbare Strahlablenkvorrichtung |
| P | Punkt auf Augenhintergrund |
| B | Beleuchtungsstrahlengang |
| D | Detektionsstrahlengang |
| OA | Optische Achse von Kamera und Laser |
| VA | Sehachse |

**Patentansprüche**

1. Fixationskontrolleinrichtung (2) für ein ophthalmologisches Gerät (1), aufweisend eine Fixierlichtquelle (4) für sichtbares Licht und eine Optik (7) zur Abbildung der Fixierlichtquelle (4) auf einen Augenhintergrund (3.1) mit einer Retina (3.3) und einer Fovea (3.2), aufweisend

   - mindestens eine Messlichtquelle (5.1, 5.2) zur Emission von Licht mindestens zweier Wellenlängen, bei denen die Fovea (3.2) eine andere Reflektivität aufweist als die übrige Retina (3.3),
   - eine Optik (7) zur Abbildung der Messlichtquelle (5.1, 5.2) auf zumindest einen Teil des Augenhintergrunds (3.1) und
   - mindestens einen Detektor (10, 10.1, 10.2) zum separaten Erfassen der Intensitäten der zwei Wellenlängen als jeweiliges Detektorsignal ($S_1$, $S_2$) nach einer Reflexion an dem Augenhintergrund (3.1).

2. Fixationskontrolleinrichtung (2) nach Anspruch 1, wobei für die zwei Wellenlängen $R_{\lambda 1,Retina}/ R_{\lambda 2,Retina} \neq R_{\lambda 1,Fove(ol)a}/ R_{\lambda 2,Fove(ol)a}$ gilt mit: $R_{\lambda 1,Retina}$ ist der Reflexionsgrad der ersten Wellenlänge bei Reflexion an der Retina (3.3), $R_{\lambda 2,Retina}$ ist der Reflexionsgrad der zweiten Wellenlänge bei Reflexion an der Retina (3.3), $R_{\lambda 1,Fove(ol)a}$ ist der Reflexionsgrad der ersten Wellenlänge bei Reflexion an der Fovea/Foveola (3.2) und $R_{\lambda 2,Fove(ol)a}$ ist der Reflexionsgrad der zweiten Wellenlänge bei Reflexion an der Fovea/Foveola (3.3).

3. Fixationskontrolleinrichtung (2) nach Anspruch 1 oder 2, **gekennzeichnet durch** eine Auswerteeinheit (11), welche, insbesondere wiederholt, ein Verhältnis der beiden Detektorsignale ($S_1$, $S_2$) ermittelt und mit einem vorgegebenen Wert oder einem vorgegebenen Wertebereich vergleicht und in Abhängigkeit des Vergleichsergebnisses ein Ergebnissignal ausgibt.

4. Fixationskontrolleinrichtung (2) nach Anspruch 1 oder 2 oder 3, **dadurch gekennzeichnet, dass** ein Strahlengang (D) von dem Augenhintergrund (3.1) zu dem mindestens einen Detektor und ein Strahlengang (B) von dem Augenhintergrund (3.1) zu der mindestens einen Messlichtquelle (4) teilweise identisch sind.

5. Fixationskontrolleinrichtung (2) nach Anspruch 1, 2 oder 3 oder 4, **gekennzeichnet durch** ein zweidimensionales Feld von Detektoren (10) mit jeweiligen Detektorsignalen und **durch** entsprechend flächige ausgedehnte Ausbildung der Messlichtquelle (5) zur Emission von ausschließlich unsichtbaren Messwellenlängen, wobei die Messlichtquelle (5) aus Sicht eines Patienten die Fixierlichtquelle (4) zumindest teilweise umgibt.

6. Fixationskontrolleinrichtung (2) nach einem der vorhergehenden Ansprüche, **gekennzeichnet durch** mehrere punktförmig in den Augenhintergrund abbildbare Messlichtquellen (5) mit jeweils zwei Wellenlängen, bei denen die Fovea eine andere Reflektivität aufweist als die übrige Retina .

7. Fixationskontrolleinrichtung (2) nach einem der vorhergehenden Ansprüche, **gekennzeichnet durch** einstellbare Projektion der Lichtquellen (4, 5) in unterschiedliche Sehentfernungen.

8. Fixationskontrolleinrichtung (2) nach einem der vorhergehenden Ansprüche, **gekennzeichnet durch** eine Fixierlichtquelle (4) auf der optischen Achse des Detektors (10) und eine andere Fixierlichtquelle (15) abseits der optischen Achse des Detektors (10).

9. Fixationskontrolleinrichtung (2) für ein ophthalmologisches Gerät (1) nach einem der vorhergehenden Ansprüche, **gekennzeichnet durch** eine Auswerteeinheit (11), welche einen Zustand einer Fixation eines Fixierziels **durch** ein Auge identifiziert und daraufhin eine Bildaufnahme von dem Auge oder eine Behandlung des Auges auslöst und/oder eine Bewegungsverfolgung des Auges einrastet.

10. Ophthalmologisches Gerät (1) mit einer Fixationskontrolleinrichtung (2) nach einem der vorhergehenden Ansprüche.

11. Verfahren zur Kontrolle einer Fixation eines Auges (3), wobei folgende Schritte durchgeführt werden:

- Beleuchten zumindest eines Teils eines Augenhintergrunds (3.1), der eine Retina (3.3) und eine Fovea (3.2) aufweist, mit Licht zweier Wellenlängen, bei denen die Fovea/Foveola (3.2) eine andere Reflektivität aufweist als die übrige Retina (3.3),
- separates Erfassen der Intensitäten der zwei Wellenlängen mittels mindestens eines Detektors (10, 10.1, 10.2) als jeweiliges Detektorsignal ($S_1$, $S_2$) nach einer Reflexion an einem Augenhintergrund (3.1),
- Ermitteln eines Verhältnisses der beiden Detektorsignale ($S_1$, $S_2$),
- Vergleichen des Verhältnisses mit einem vorgegebenen Wert oder einem vorgegebenen Wertebereich und
- Ausgeben eines Ergebnissignals in Abhängigkeit des Vergleichsergebnisses.

12. Betriebsverfahren für ein ophthalmologisches Gerät nach Anspruch 1, **dadurch gekennzeichnet, dass** identifiziert wird, dass ein Auge ein Fixierziel fixiert und daraufhin eine Bildaufnahme von dem Auge oder eine Behandlung des Auges ausgelöst und/oder eine Bewegungsverfolgung des Auges eingerastet wird.

13. Verfahren nach einem der vorhergehenden Verfahrens-Ansprüche, **gekennzeichnet durch** Intensitätsmodulation der mindestens einen Messlichtquelle (5, 5.1, 5.2)

a) mit für die beiden Wellenlängen unterschiedlichen Frequenzen und Erfassung der reflektierten Wellenlängen in einem gemeinsamen Detektor (10), wobei anschließend eine elektronische Trennung in die Detektorsignale ($S_1$, $S_2$) erfolgt, oder

b) mit für die beiden Wellenlängen identischer Frequenz bei versetzten Phasen und phasensensitiver Erfassung der beiden Wellenlängen in einem gemeinsamen Detektor (10).

14. Verfahren nach einem der vorhergehenden Verfahrens-Ansprüche, **gekennzeichnet durch** gepulsten Betrieb der mindestens einen Messlichtquelle (5.1, 5.2).

**15.** Fixationskontrolleinrichtung (2) nach einem der Ansprüche 1 bis 9, **dadurch gekennzeichnet, dass** eine Steuereinheit (11) die mindestens eine Messlichtquelle (5, 5.1, 5.2) intensitätsmoduliert

a) mit für die beiden Wellenlängen unterschiedlichen Frequenzen und Erfassung der reflektierten Wellenlängen in einem gemeinsamen Detektor (10), wobei anschließend eine elektronische Trennung in die Detektorsignale ($S_1$, $S_2$) erfolgt,
oder
b) mit für die beiden Wellenlängen identischer Frequenz bei versetzten Phasen und phasensensitiver Erfassung der beiden Wellenlängen in einem gemeinsamen Detektor (10).

**16.** Fixationskontrolleinrichtung (2) nach einem der Ansprüche 1 bis 9 oder 15,
**dadurch gekennzeichnet, dass** eine Steuereinheit (11) die mindestens eine Messlichtquelle (5.1, 5.2) gepulst betreibt.

## Claims

**1.** Fixation control apparatus (2) for an ophthalmological device (1), comprising a fixation light source (4) for visible light and an optical assembly (7) for imaging the fixation light source (4) on an ocular fundus (3.1) with a retina (3.3) and a fovea (3.2), comprising

- at least one measurement light source (5.1, 5.2) for emitting light with at least two wavelengths, in the case of which the fovea (3.2) has a different reflectivity to the rest of the retina (3.3),
- an optical assembly (7) for imaging the measurement light source (5.1, 5.2) on at least one part of the ocular fundus (3.1) and
- at least one detector (10, 10.1, 10.2) for separate acquisition of the intensities of the two wavelengths as respective detector signal ($S_1$, $S_2$) after reflection at the ocular fundus (3.1).

**2.** Fixation control apparatus (2) according to Claim 1, wherein the following applies to the two wavelengths : $R_{\lambda 1,retina}/R_{\lambda 2,retina} \neq R_{\lambda 1,fove(ol)a}/R_{\lambda 2,fove(ol)a}$, where: $R_{\lambda 1,retina}$ is the reflectance of the first wavelength in the case of reflection at the retina (3.3), $R_{\lambda 2,retina}$ is the reflectance of the second wavelength in the case of reflection at the retina (3.3), $R_{\lambda 1,fove(ol)a}$ is the reflectance of the first wavelength in the case of reflection at the fovea/foveola (3.2) and $R_{\lambda 2,fove(ol)a}$ is the reflectance of the second wavelength in the case of reflection at the fovea/foveola (3.3).

**3.** Fixation control apparatus (2) according to Claim 1 or 2, **characterized by** an evaluation unit (11) which, in particular repeatedly, establishes a ratio of the two detector signals ($S_1$, $S_2$) and compares said ratio to a predetermined value or a predetermined value range and outputs a result signal depending on the comparison result.

**4.** Fixation control apparatus (2) according to Claim 1 or 2 or 3, **characterized in that** a beam path (D) from the ocular fundus (3.1) to the at least one detector and a beam path (B) from the ocular fundus (3.1) to the at least one measurement light source (4) are at least partly identical.

**5.** Fixation control apparatus (2) according to Claim 1,2 or 3 or 4, **characterized by** a two-dimensional field of detectors (10) with respective detector signals and by a correspondingly planar stretched embodiment of the measurement light source (5) for emitting only invisible measurement wavelengths, wherein, from the view of a patient, the measurement light source (5) at least partly surrounds the fixation light source (4).

**6.** Fixation control apparatus (2) according to one of the preceding claims, **characterized by** a plurality of punctiform measurement light sources (5) imageable in the ocular fundus, each comprising two wavelengths at which the fovea has a different reflectivity than the rest of the retina.

**7.** Fixation control apparatus (2) according to one of the preceding claims, **characterized by** an adjustable projection of the light sources (4, 5) at different viewing distances.

**8.** Fixation control apparatus (2) according to one of the preceding claims, **characterized by** a fixation light source (4) on the optical axis of the detector (10) and another fixation light source (15) off the optical axis of the detector (10).

9. Fixation control apparatus (2) for an ophthalmological device (1) according to one of the preceding claims, **characterized by** an evaluation unit (11), which identifies a state of a fixation of a fixation target by an eye and thereupon triggers an image recording of the eye or a treatment of the eye and/or engages into movement tracking of the eye.

10. Ophthalmological device (1) comprising a fixation control apparatus (2) according to one of the preceding claims.

11. Method for controlling a fixation of an eye (3), wherein the following steps are carried out:

- illuminating at least part of an ocular fundus (3.1), which includes a retina (3.3) and a fovea (3.2), with light of two wavelengths, where the fovea/foveola (3.2) has a different reflectivity to the rest of the retina (3.3),
- separate acquisition of the intensities of the two wavelengths as respective detector signal ($S_1$, $S_2$) by means of at least one detector (10, 10.1, 10.2) after a reflection at an ocular fundus (3.1),
- establishing a ratio of the two detector signals ($S_1$, $S_2$),
- comparing the ratio with a predetermined value or a predetermined value range, and
- outputting a result signal depending on the comparison result.

12. Operating method for an ophthalmological device according to Claim 1, **characterized in that** an identification is carried out in respect of an eye fixating a fixation target and whereupon an image recording of the eye or a treatment of the eye is triggered and/or a movement tracking of the eye is engaged into.

13. Method according to one of the preceding method claims, **characterized by** an intensity modulation of the at least one measurement light source (5, 5.1, 5.2)

a) with frequencies that are different for both wavelengths and with acquisition of the reflected wavelengths in a common detector (10), with there subsequently being an electronic separation into the detector signals ($S_1$, $S_2$) or
b) with identical frequencies for the two wavelengths in the case of offset phases and with phase-sensitive acquisition of the two wavelengths in a common detector (10).

14. Method according to one of the preceding method claims, **characterized by** a pulsed operation of the at least one measurement light source (5.1, 5.2).

15. Fixation control apparatus (2) according to one of Claims 1 to 9, **characterized in that** a control unit (11) performs intensity modulation of the at least one measurement light source (5, 5.1, 5.2)

a) with frequencies that are different for the two wavelengths and with acquisition of the reflected wavelengths in a common detector (10), with there subsequently being an electronic separation into the detector signals ($S_1$, $S_2$) or
b) with identical frequencies for the two wavelengths in the case of offset phases and with phase-sensitive acquisition of the two wavelengths in a common detector (10).

16. Fixation control apparatus (2) according to one of Claims 1 to 9 or 15, **characterized in that** a control unit (11) operates the at least one measurement light source (5.1, 5.2) in a pulsed manner.

**Revendications**

1. Dispositif de contrôle de la fixation (2) destiné à un appareil ophtalmologique (1), comprenant une source de lumière de fixation (4) pour lumière visible et une optique (7) destinée à former l'image de la source de lumière de fixation (4) sur un fond d'oeil (3.1) comportant une rétine (3.3) et une fovéa (3.2), comprenant

- au moins une source de lumière de mesure (5.1, 5.2) destinée à émettre une lumière ayant au moins deux longueurs d'onde auxquelles la fovéa (3.2) présente une réflectivité différente de celle du reste de la rétine (3.3),
- une optique (7) destinée à former l'image de la source de lumière de mesure (5.1, 5.2) sur au moins une partie du fond d'oeil (3.1) et
- au moins un détecteur (10, 10.1, 10.2) destiné à détecter séparément les intensités des deux longueurs d'onde sous la forme d'un signal de détecteur respectif ($S_1$, $S_2$) après une réflexion sur le fond d'oeil (3.1).

**2.** Dispositif de contrôle de la fixation (2) selon la revendication 1, dans lequel, pour les deux longueurs d'onde, on a $R_{\lambda1,Rétine}/R_{\lambda2,Rétine} \neq R_{\lambda1,Fové(ol)al}/R_{\lambda2,Fové(ol)a}$, où : $R_{\lambda1,Rétine}$ est le coefficient de réflexion de la première longueur d'onde lors de la réflexion sur la rétine (3.3), $R_{\lambda2,Rétine}$ est le coefficient de réflexion de la seconde longueur d'onde lors de la réflexion sur la rétine (3.3), $R_{\lambda1,Fové(ol)al}$ est le coefficient de réflexion de la première longueur d'onde lors de la réflexion sur la fovéa/fovéola (3.2) et $R_{\lambda2,Fove(ol)a}$ est le coefficient de réflexion de la seconde longueur d'onde lors de la réflexion sur la fovéa/fovéola (3.3).

**3.** Dispositif de contrôle de la fixation (2) selon la revendication 1 ou 2, **caractérisé par** une unité d'évaluation (11) qui détermine, notamment de manière répétée, un rapport des deux signaux de détecteur ($S_1$, $S_2$) et le compare à une valeur prédéterminée ou à une gamme de valeurs prédéterminée et délivre un signal de résultat en fonction du résultat de la comparaison.

**4.** Dispositif de contrôle de la fixation (2) selon la revendication 1 ou 2 ou 3, **caractérisé en ce qu'**un trajet de faisceau (D) du fond d'oeil (3.1) à l'au moins un détecteur et un trajet de faisceau (B) du fond d'oeil (3.1) à l'au moins une source de lumière de mesure (4) sont partiellement identiques.

**5.** Dispositif de contrôle de la fixation (2) selon la revendication 1, 2 ou 3 ou 4, **caractérisé par** un champ bidimensionnel de détecteurs (10) ayant des signaux de détecteurs respectifs et par la formation correspondante d'une image de grande étendue de la source de lumière de mesure (5) pour l'émission de longueurs d'onde exclusivement invisibles, dans lequel la source de lumière de mesure (5), telle que la voit un patient, entoure au moins partiellement la source de lumière de fixation (4).

**6.** Dispositif de contrôle de la fixation (2) selon l'une quelconque des revendications précédentes, **caractérisé par** une pluralité de sources de lumière de mesure ponctuelles (5) dont les images peuvent être formées dans le fond d'oeil ayant respectivement deux longueurs d'onde auxquelles la fovéa présente une réflectivité différente de celle du reste de la rétine.

**7.** Dispositif de contrôle de la fixation (2) selon l'une quelconque des revendications précédentes, **caractérisé par** la projection réglable des sources de lumière (4, 5) à différentes distances de vision.

**8.** Dispositif de contrôle de la fixation (2) selon l'une quelconque des revendications précédentes, **caractérisé par** une source de lumière de fixation (4) sur l'axe optique du détecteur (10) et par une autre source de lumière de fixation (15) en dehors de l'axe optique du détecteur (10).

**9.** Dispositif de contrôle de la fixation (2) destiné à un appareil ophtalmologique (1) selon l'une quelconque des revendications précédentes, **caractérisé par** une unité d'évaluation (11) qui identifie un état de fixation d'une cible de fixation par un oeil et déclenche alors une acquisition d'image de l'oeil ou un traitement de l'oeil et/ou provoque une poursuite du mouvement de l'oeil.

**10.** Appareil ophtalmologique (1) comportant un dispositif de contrôle de la fixation (2) selon l'une quelconque des revendications précédentes.

**11.** Procédé de contrôle de la fixation d'un oeil (3), dans lequel les étapes suivantes sont exécutées :

- éclairer au moins une partie d'un fond d'oeil (3.1) qui comprend une rétine (3.3) et une fovéa (3.2), avec une lumière ayant deux longueurs d'onde auxquelles la fovéa/fovéola (3.2) présente une réflectivité différente de celle du reste de la rétine (3.3),
- détecter séparément les intensités des deux longueurs d'onde au moyen d'au moins un détecteur (10, 10.1, 10.2) sous la forme d'un signal de détecteur respectif ($S_1$, $S_2$) après une réflexion sur un fond d'oeil (3.1),
- déterminer un rapport des deux signaux de détecteur ($S_1$, $S_2$),
- comparer le rapport à une valeur prédéterminée ou à une gamme de valeurs prédéterminée et
- délivrer un signal de résultat en fonction du résultat de la comparaison.

**12.** Procédé de mise en fonctionnement destiné à un appareil ophtalmologique selon la revendication 1, **caractérisé en ce qu'**on identifie qu'un oeil fixe une cible de fixation et qu'une acquisition d'image de l'oeil ou qu'un traitement de l'oeil est alors déclenché et/ou qu'une poursuite du mouvement de l'oeil est alors provoquée.

**13.** Procédé selon l'une quelconque des revendications de procédé précédentes, **caractérisé par** la modulation d'intensité de l'au moins une source de lumière de mesure (5, 5.1, 5.2)

a) à des fréquences différentes pour les deux longueurs d'onde et avec détection des longueurs d'onde réfléchies dans un détecteur commun (10), dans lequel une séparation électronique est ensuite effectuée dans les signaux de détecteur ($S_1$, $S_2$), ou

b) à une fréquence identique pour les deux longueurs d'onde en cas de phases décalées et avec détection sensible à la phase des deux longueurs d'onde dans un détecteur commun (10).

**14.** Procédé selon l'une quelconque des revendications de procédé précédentes, **caractérisé par** un fonctionnement pulsé de l'au moins une source de lumière de mesure (5.1, 5.2).

**15.** Dispositif de contrôle de la fixation (2) selon l'une quelconque des revendications 1 à 9, **caractérisé en ce qu'**une unité de commande (11) module en intensité l'au moins une source de lumière de mesure (5, 5.1, 5.2)

a) à des fréquences différentes pour les deux longueurs d'onde et avec détection des longueurs d'onde réfléchies dans un détecteur commun (10), dans lequel une séparation électronique est ensuite effectuée dans les signaux de détecteur ($S_1$, $S_2$), ou

b) à une fréquence identique pour les deux longueurs d'onde en cas de phases décalées et avec détection sensible à la phase des deux longueurs d'onde dans un détecteur commun (10).

**16.** Dispositif de contrôle de la fixation (2) selon l'une quelconque des revendications 1 à 9 ou 15, **caractérisé en ce qu'**une unité de commande (11) fait fonctionner de manière pulsée l'au moins une source de lumière de mesure (5.1, 5.2).

Fig. 1

Fig. 2

EP 2 445 387 B1

Fig. 3

Fig. 4

17

Fig. 5

Fig. 6

Fig. 7

Fig. 8

Fig. 9

Fig. 10

20

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20060142742 A1
- US 6027216 A
- US 2004252277 A1
- US 2008084539 A1